# EUROPEAN PATENT APPLICATION

(11) **EP 4 692 349 A1**
(43) Date of publication of application: **11.02.2026**
(21) Application number: 24785112.4
(22) Date of filing: 29.03.2024
(51) Int. Cl.: C12N 15/113, C12N 15/63

(54) **MRNA 3'UTR SEQUENCE FOR INDUCING INCREASED MRNA TRANSLATION**

(30) Priority: 03.04.2023 KR 20230043659
(71) Applicant: Korea Advanced Institute of Science and Technology, Daejeon 34141 (KR); Korea University Research and Business Foundation, Seoul 02841 (KR)
(72) Inventor: KIM, Yoon Ki, Daejeon 34141 (KR); SHIN, Min-Kyung, Seoul 02463 (KR)
(74) Representative: Dragotti & Associati S.P.A.
(86) International application number: PCT/KR2024/004007
(87) International publication number: WO 2024/210405

(57) **Abstract**

When proteins are synthesized from mRNA, various factors determine the efficiency of protein synthesis from mRNA. The efficiency of mRNA translation can be determined by various factors, including the activity levels of intracellular translation factors, the nucleotide sequences and RNA structures present in mRNA, and the extent of RNA modifications. The present invention relates to a new nucleotide sequence that enhances the efficiency of mRNA translation. Specifically, when a U-rich nucleotide sequence is present after the translation termination codon, it more efficiently recruits translation factors such as eIF4G, eRF1, and eRF3, thereby inducing an increase in protein translation.

## Description

### Technical Field

The present invention relates to an mRNA 3'UTR sequence that induces increased mRNA translation, specifically a nucleotide that increases mRNA stability and protein translation efficiency by including a uridine sequence in the 3' UTR (Untranslated region), a method for increasing translation efficiency, and a vector containing the same.

### Background Art

Protein synthesis in eukaryotic cells is primarily induced by the cap structure present at the 5' end of mRNA. Many intracellular factors are involved in attracting the ribosome to the 5'-cap structure of mRNA, ultimately inducing the initiation of protein translation. Since mRNA vaccines have recently been widely used as vaccines against coronaviruses, extensive research and development has been focused on the development of disease therapeutics using mRNA. The current focus is on developing technologies to enhance mRNA stability, thereby increasing the amount of protein ultimately expressed. However, even with mRNA of the same stability, higher protein translation efficiency would allow for the production of more protein, thereby enhancing its utility as a disease therapeutic or vaccine.

NMD is a representative mechanism among various gene expression regulation mechanisms, particularly involved in mRNA quality control, with Nonsense-mediated mRNA Decay (NMD) being well-known. The NMD mechanism recognizes mRNA containing abnormal protein termination codons (Premature termination codons; PTCs) and ultimately removes them from the cell. If mRNA containing a PTC is not recognized by the NMD mechanism, it will express a protein that is shorter than the normal-length protein. If the resulting short protein possesses a dominant-negative effect, it can inhibit the function of the normal protein, thereby impairing cellular function. In other words, the NMD mechanism can be viewed as a quality control mechanism that preemptively recognizes and removes abnormal short proteins that could harm the cell at the mRNA level. It is estimated that approximately 30% of all reported genetic diseases are caused by PTCs. Most PTCs formed on mRNA are believed to be closely associated with the NMD mechanism. Furthermore, recent reports have accumulated on the association between the NMD mechanism and carcinogenic processes. Research on the mechanisms of NMD is being conducted in numerous laboratories worldwide to develop treatments for various genetic diseases and cancers.

### Technical Problem

An object of the present invention is to provide a nucleotide containing uridine in a 3' UTR (Untranslated Region) after a protein translation termination codon of a desired nucleic acid sequence.

Another objective of the present invention is to provide a method of increasing the protein translation efficiency of the desired nucleic acid, including the step of inserting a uridine into a 3' UTR after a protein translation termination codon of the desired nucleic acid sequence.

Another objective of the present invention is to provide a vector for increasing the translation efficiency of the protein, comprising a sequence encoding the protein and including uridine (uridine) in the 3' UTR following the protein translation termination codon of the sequence encoding the protein.

However, the technical problem to be achieved by the present invention is not limited to the above-mentioned problems, and other problems that are not mentioned will be clearly understood by a skilled person in the art from the following description.

### Solution to Problem

Various embodiments of the present invention are described with reference to the accompanying drawings. In the following description, various specific details, such as specific forms, compositions, and processes, are described for a complete understanding of the present invention. However, specific embodiments may be implemented without one or more of these specific details or in combination with other known methods and forms. In other examples, known processes and manufacturing techniques are not described in detail to avoid making the present invention unnecessary or ambiguous. A reference throughout this specification to a single embodiment means that the special features, forms, compositions, or characteristics described in connection with that embodiment are included in one or more embodiments of the present invention. Therefore, the situations of embodiments described at various locations throughout this specification do not necessarily represent the same embodiments of the invention. Additionally, special features, forms, compositions, or characteristics may be combined in any suitable manner in one or more embodiments.

Unless otherwise defined in this specification, all scientific and technical terms used in this specification have the same meanings as those commonly understood by those skilled in the art to which the present invention pertains.

In the present invention, a reporter mRNA was artificially created by introducing 10 random sequences into the 3'UTR of a specific reporter mRNA that is a target of nonsense-mediated mRNA decay (NMD). Specifically, a reporter was artificially created by inserting 4¹⁰ different nucleotide sequences immediately following the premature translation termination codon that triggers NMD, and this was introduced into HeLa cell lines. Subsequently, we identified nucleotide sequences that influence mRNA stability using next-generation sequencing techniques. As a result, we discovered that when 10 uridines (U10) are present immediately after the premature translation termination codon, the reporter mRNA becomes highly stable. Since NMD is directly related to protein translation, we investigated the effect of the U10 sequence found above on protein translation. The results showed that the U10 sequence immediately following the protein translation termination codon increases protein translation efficiency. The U10 sequence immediately following the protein translation termination codon was confirmed to directly influence the translation termination process rather than the initiation or elongation processes of protein translation. The present invention relates to a method for increasing the translation efficiency of mRNA, which involves inserting a uridine-rich sequence (U-rich sequence) immediately following the protein translation termination codon to increase protein expression levels.

In one embodiment of the present invention, the distance between the protein translation termination codon and the uridine in the target nucleic acid sequence is 120 nt or less. Preferably, the distance between the protein translation termination codon and the uridine in the target nucleic acid sequence is 105 nt or less, more preferably 90 nt or less, but is not limited thereto.

In one embodiment of the present invention, nucleotides are provided wherein the length of the uridine is 6 nt or more. Preferably, the length of the uridine is 10 nt or more, but this is not limited thereto.

In the present invention, the term "nucleic acid" refers to DNA (deoxyribonucleic acid) and RNA (ribonucleic acid). It is used synonymously with the term "polynucleotide". Preferably, it is a polymer composed of nucleotide monomers covalently linked by phosphodiester bonds in a sugar/phosphate backbone. Alternatively, it may include DNA or RNA with modified bases, modified sugars, or modified backbones. However, considering the purpose of the present invention, it is preferable that the nucleic acid in the present invention refers to RNA, particularly mRNA. As a related concept, the nucleic acid intended for expression in the present invention may correspond to a target sequence intended for expression, and may be a target mRNA sequence for which translation is desired to be increased using methods such as those for increasing protein translation efficiency in the present invention.

In the present invention, the term "artificial nucleic acid" may be understood as a non-natural nucleic acid molecule that does not naturally exist in nature. The artificial nucleic acid may be composed of 100% of a sequence that does not exist in nature, or may be composed by mixing a part of a sequence (wild type) that exists in nature and a part of a sequence that does not exist in nature. When composed solely of sequences existing in nature, the sequences may be a mixture of sequences derived from different species. When a part of a sequence existing in the natural world and a part of a sequence not existing in the natural world are mixed and formed, the sequence existing in the natural world may be a mixture of sequences derived from one or more species. Artificial nucleic acids may be non-natural due to naturally occurring modifications of their individual sequences, e.g., structural modifications of naturally occurring nucleotides. Additionally, artificial nucleic acids may be DNA molecules, RNA molecules, or hybrid molecules containing DNA and RNA components. The nucleic acid used for stabilizing the mRNA or increasing the translation of the mRNA, in particular, may be an artificial nucleic acid obtained by artificially modifying a sequence derived from a single species, or a mixture of sequences derived from different species. Such artificial nucleic acids may be understood as a type of vector. In the context of the present invention, a vector may include an artificial nucleic acid selected for nucleic acid stabilization. Such vectors may include storage vectors, expression vectors, cloning vectors, and transport vectors. A storage vector is a vector that allows for the convenient storage of nucleic acid molecules, such as mRNA molecules. An expression vector can be used for the production of expression products, such as RNA (e.g., mRNA), peptides, polypeptides, or proteins. Cloning vectors are vectors that typically include a cloning site that can be used to insert nucleic acid sequences into the vector. Cloning vectors may include, for example, plasmid vectors or bacteriophage vectors. A transport vector is a vector suitable for transporting nucleic acid molecules into cells or organisms, such as a viral vector. In the context of the present invention, a vector may be, for example, an RNA vector or a DNA vector. Preferably, the vector of the present invention is a plasmid vector or a viral vector, but is not limited thereto. The vector can be used to introduce the desired DNA or RNA (e.g., mRNA) nucleic acid into cells, preferably into eukaryotic cells, thereby altering the original characteristics of the target organism. This process is referred to as transfection. The transfection may be performed by any method known to those skilled in the art for introducing nucleic acid molecules into cells, preferably into eukaryotic cells such as mammalian cells. Such methods include, for example, electroporation, lipofection based on cationic lipids and/or liposomes, calcium phosphate precipitation, nanoparticle-based transfection, virus-based transfection, or transfection using cationic polymers such as DEAE-dextran or polyethyleneimine. Alternatively, the transformation may be performed using a virus, such as a lentivirus. The introduced target entity whose original traits have been altered by the methods is referred to as a transformant.

In this specification, the terms "host cell" or "cell line" include eukaryotic and prokaryotic organisms and refer to any genetically transformable organism capable of replicating the vector or expressing the gene encoded by the vector. Host cells can be transfected or transformed by the vector, which refers to the process of introducing exogenous nucleic acid molecules into the host cells.

The host cells of the present invention may preferably include bacterial cells, CHO cells, HeLa cells, HEK293 cells, BHK-21 cells, COS7 cells, COP5 cells, A549 cells, NIH3T3 cells, etc., but are not limited thereto.

The nucleotides, methods for increasing translation efficiency, and vectors containing the same, which increase the stability of mRNA and protein translation efficiency, may be used for the purpose of enhancing immunity in the target organism. The immunity-boosting refers to the amplification of the desired immune response against selected antigens, such as characteristic components of bacterial surfaces, viral particles, or tumor antigens. This can be induced naturally or artificially, and in the case of artificial immune response amplification, vaccine administration is the most common method. A vaccine is typically understood as a preventive or therapeutic substance that provides at least one antigen, preferably an immunogen. Antigens or immunogens can be derived from any material suitable for vaccination. For example, antigens or immunogens can be derived from bacteria or viral particles, or from tumors or cancerous tissues. Antigens or immunogens stimulate the adaptive immune system within the body. Traditionally, vaccines primarily utilized proteins or peptides derived from antigens or immunogens as stimuli for the adaptive immune system. However, recent efforts have focused on using nucleic acids as stimuli. In particular, mRNA has gained attention in the healthcare field as a new therapeutic method due to its advantage of being able to express the desired protein temporarily in the cytoplasm without needing to enter the nucleus, unlike DNA. For example, in the case of emerging infectious diseases, rapid vaccine production is facilitated by simply altering the nucleotide sequence of mRNA. Additionally, the mRNA used as a vaccine is rapidly degraded after expressing proteins within human cells, offering superior safety compared to DNA vaccines. However, mRNA has structural instability compared to DNA, limiting its industrial application. In the case of mRNA vaccines first attempted for COVID-19, the half-life was approximately 4 hours, making it unstable, and approximately 30-100 µg of mRNA administration was required per dose to achieve sufficient protein expression. Such single-dose administration of large amounts of mRNA became a cause of various side effects depending on the individual. Therefore, from the viewpoint of solving these conventional problems, nucleotides, vectors, or artificial nucleic acids that increase the stability and protein translation efficiency of mRNAs of the present invention can be administered as vaccines containing antigens or nucleic acids derived from immunogens suitable for desired immune enhancement. In such cases, the uridine sequence present in the 3' UTR is preferably located on the 3' side of the protein translation stop codon of the antigen genetic information in the nucleic acid vaccine. Additionally, this enhances the stability of the nucleic acid vaccine and enables efficient synthesis of the antigen within the body, thereby allowing high efficacy in disease treatment and prevention even with a small vaccine dose. Furthermore, since the vaccine dose required to achieve the desired therapeutic effect can be reduced, this is expected to lower vaccine production costs. The vaccine may additionally comprise an adjuvant (component) to enhance the expected effects. An adjuvant is a pharmacological and/or immunological agent that can modify or enhance the effects of other formulations, such as drugs or vaccines. This term is broadly interpreted and encompasses a wide range of substances. Typically, these substances can enhance the immunogenicity of antigens. For example, adjuvants can be recognized by the innate immune system and induce an innate immune response. The artificial sequences of the present invention, which include nucleotides that enhance the stability of mRNA and the efficiency of protein translation, or the vaccine, can have infinitely expandable applications depending on the type of antigen or immunogen included. For example, when the antigen or immunogen includes virus-derived nucleic acids, the vaccine is a vaccine for the corresponding virus. For instance, when the antigen or immunogen is derived from the SARS-CoV-2 virus, the vaccine is a vaccine for coronavirus disease (COVID-19). If the antigen or immunogen is derived from an influenza virus (Influenza virus A, B, or C), the vaccine is an influenza vaccine. If the antigen or immunogen is derived from a papillomavirus, the vaccine is a cervical cancer vaccine.

The nucleotides, methods for increasing translation efficiency, and vectors containing them that enhance the stability of the mRNA and the protein translation efficiency of the present invention can be used as a pharmaceutical composition for preventing or treating the target disease by enhancing the immunity of the target organism.

The nucleotides, methods for increasing translation efficiency, and vectors containing them, which increase the stability and protein translation efficiency of the mRNA of the present invention, can be used as a pharmaceutical composition for the prevention or treatment of various genetic diseases and disorders caused by quantitative deficiencies of specific mRNA or specific proteins.

The nucleotides that enhance the stability of the mRNA and the protein translation efficiency, the method for enhancing the translation efficiency, and the vector containing the same of the present invention can be used as an alternative to existing DNA-based disease treatment methods.

In the present invention, the "treatment" or "amelioration" may include, without limitation, any act capable of ameliorating or beneficially altering symptoms caused by a disease, using the composition of the present invention.

Furthermore, without being limited thereto, the methods for preventing or treating the diseases may include combination therapy that further comprises administering a compound or material having therapeutic activity against one or more diseases.

In the present invention, the term "combination therapy" should be understood to mean simultaneous, individual, or sequential administration. In the case of sequential or individual administration, the interval between the administration of the second component should be such that the beneficial effects of the combination therapy are not lost.

In the present invention, the pharmaceutical composition may be characterized by being in the form of capsules, tablets, granules, injections, ointments, powders, or beverages, and the pharmaceutical composition may be characterized by being targeted to humans.

The pharmaceutical composition of the present invention may be formulated in the form of oral preparations such as powders, granules, capsules, tablets, and aqueous suspensions, preparations for external use, suppositories, and sterile injectable solutions, respectively, according to conventional methods, and used. However, the pharmaceutical composition is not limited thereto. The pharmaceutical composition of the present invention may further comprise a pharmaceutically acceptable carrier. As the pharmaceutically acceptable carrier, a binder, a glidant, a disintegrant, an excipient, a solubilizer, a dispersant, a stabilizer, a suspending agent, a pigment, a flavor, and the like may be used for oral administration; a buffer, a preserving agent, a pain-relieving agent, a solubilizer, an isotonic agent, a stabilizer, and the like may be used in admixture for injections; and a base, an excipient, a lubricant, a preserving agent, and the like may be used for topical administration. The preparations of the pharmaceutical composition of the present invention may be prepared in various ways by being mixed with the pharmaceutically acceptable carrier as described above. For example, for oral administration, the pharmaceutical composition may be formulated in the form of tablets, troches, capsules, elixirs, suspensions, syrups, wafers, or the like. For injections, the pharmaceutical composition may be formulated in the form of unit dosage ampoules or multiple dosage forms. Alternatively, the pharmaceutical composition may be formulated into solutions, suspensions, tablets, capsules, sustained-release preparations, or the like.

Meanwhile, as examples of carriers, diluents, or excipients suitable for making preparations, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, or the like may be used. In addition, a filler, an anti-coagulant, a lubricant, a wetting agent, a fragrance, an emulsifier, a preservative, and the like may further be included.

The route of administration of the pharmaceutical composition according to the present invention includes, but is not limited to, oral, intravenous, intramuscular, intraarterial, intramedullary, intradural, intracardiac, transdermal, subcutaneous, intraperitoneal, intranasal, intestinal, topical, sublingual, or rectal route. Oral or parenteral administration is preferred.

In the present invention, the "parenteral" includes subcutaneous, intradermal, intravenous, intramuscular, intraarticular, intrabursal, intrasternal, intradural, intralesional, and intracranial injection or infusion techniques. The pharmaceutical composition of the present invention may also be administered in the form of suppositories for rectal administration.

The pharmaceutical composition of the present invention may vary depending on a variety of factors, including activity of a certain compound used, the patient's age, body weight, general health status, sex, diet, frequency of administration, route of administration, rate of excretion, drug combination, and severity of a certain disease to be prevented or treated. A dose of the pharmaceutical composition may vary depending on the patient's condition, body weight, severity of disease, drug form, route of administration, and duration, and may be appropriately selected by those skilled in the art. The pharmaceutical composition may be administered in an amount of 0.0001 to 50 mg/kg or 0.001 to 50 mg/kg, per day. Administration may be made once a day or several times a day. The dose is not intended to limit the scope of the invention in any way. The pharmaceutical composition according to the present invention may be formulated in the form of pills, sugar-coated tablets, capsules, liquids, gels, syrups, slurries, or suspensions.

In the first embodiment of the present invention, a nucleotide comprising a uridine-rich sequence in the 3' UTR (untranslated region) following the protein translation stop codon of the target nucleic acid sequence is provided.

In the second embodiment of the present invention, the nucleotide in which the target nucleic acid is an mRNA sequence is provided.

In the third embodiment of the present invention, the nucleotide in which the distance between the protein translation termination codon and the uridine-rich sequence of the target nucleic acid sequence is 120 nt or less in any one of the first to second embodiments is provided.

In the fourth embodiment of the present invention, the nucleotide in which the distance between the protein translation termination codon and the uridine-rich sequence of the target nucleic acid sequence is 105 nt or less in any one of the first to third embodiments is provided.

In the fifth embodiment of the present invention, the nucleotide in which the length of the uridine-rich sequence is 6 nt or more in any one of the first to fourth embodiments is provided.

In the sixth embodiment of the present invention, the nucleotide in which the length of the uridine-rich sequence is 10 nt or more in any one of the first to fifth embodiments is provided.

In the seventh embodiment of the present invention, the nucleotide that increases the protein translation efficiency of an RNA vaccine or therapeutic mRNA in any one of the first to sixth embodiments is provided.

In the eighth embodiment of the present invention provides a method of increasing the protein translation efficiency of a desired nucleic acid, including inserting a uridine-rich sequence into a 3' UTR after a protein translation termination codon of a desired nucleic acid sequence.

In the nineth embodiment of the present invention, the method provides a method for increasing the protein translation efficiency of the desired nucleic acid, wherein the desired nucleic acid is an mRNA sequence in the eighth embodiment.

In the tenth embodiment of the present invention, a method for increasing the protein translation efficiency of the desired nucleic acid is provided, wherein the distance between the protein translation termination codon of the desired nucleic acid sequence and the uridine-rich sequence is 120 nt or less in any one of the eighth to nineth embodiments.

In the eleventh embodiment of the present invention, a method for increasing the protein translation efficiency of the desired nucleic acid is provided, wherein the distance between the protein translation termination codon of the desired nucleic acid sequence and the uridine-rich sequence is 105 nt or less in any one of the eighth to tenth embodiments.

In the twelfth embodiment of the present invention, a method for increasing the protein translation efficiency of the desired nucleic acid is provided, wherein the length of the uridine-rich sequence is 6 nt or more in any one of the eighth to eleventh embodiments.

In the thirteenth embodiment of the present invention, a method for increasing the protein translation efficiency of the desired nucleic acid is provided, wherein the length of the uridine-rich sequence is 10 nt or more in any one of the eighth to twelfth embodiments.

In the fourteenth embodiment of the present invention, the method provides a method for increasing the protein translation efficiency of the target nucleic acid, wherein the method is for increasing the protein translation efficiency of an RNA vaccine or therapeutic mRNA.

In the fifteenth embodiment of the present invention, a vector for increasing translation efficiency of a desired protein is provided, including a sequence encoding the desired protein and a protein translation termination codon of the sequence encoding the desired protein, followed by uridine-rich sequence in the 3' UTR.

In the sixteenth embodiment of the present invention, a vector for increasing translation efficiency of a desired protein is provided, in which the distance between the protein translation termination codon and the uridine-rich sequence of the sequence encoding the desired protein is 120 nt or less in the fifteenth embodiment.

In the seventeenth embodiment of the present invention, a vector for increasing translation efficiency of a desired protein is provided, in which the distance between the protein translation termination codon and the uridine-rich sequence of the sequence encoding the desired protein is 105 nt or less in any one of the fifteenth to sixteenth embodiments.

In the eighteenth embodiment of the present invention, a vector for increasing the translation efficiency of the target protein is provided, wherein the length of the uridine-rich sequence is 6 nt or more in any one of the fifteenth to seventeenth embodiments.

In the nineteenth embodiment of the present invention, a vector for increasing the translation efficiency of the target protein is provided, wherein the length of the uridine-rich sequence is 10 nt or more in any one of the fifteenth to eighteenth embodiments.

In the twentieth embodiment of the present invention, the vector is a plasmid vector or a viral vector, and provides a vector for increasing the translation efficiency of the target protein in any one of the fifteenth to nineteenth embodiments.

The present invention will now be described in detail with reference to the embodiments.

### Advantageous Effects of Invention

To use mRNA produced *in vitro* for therapeutic purposes, the mRNA must be stable and a large amount of protein must be produced from the mRNA. The protein translation efficiency enhancement technology using U-rich sequences proposed in the present invention has the significant advantage of being easily applicable through modification of the nucleotide sequence. Therefore, it is expected to have great utility in the development of mRNA-based vaccines and therapeutic technologies.

### Brief Description of Drawings

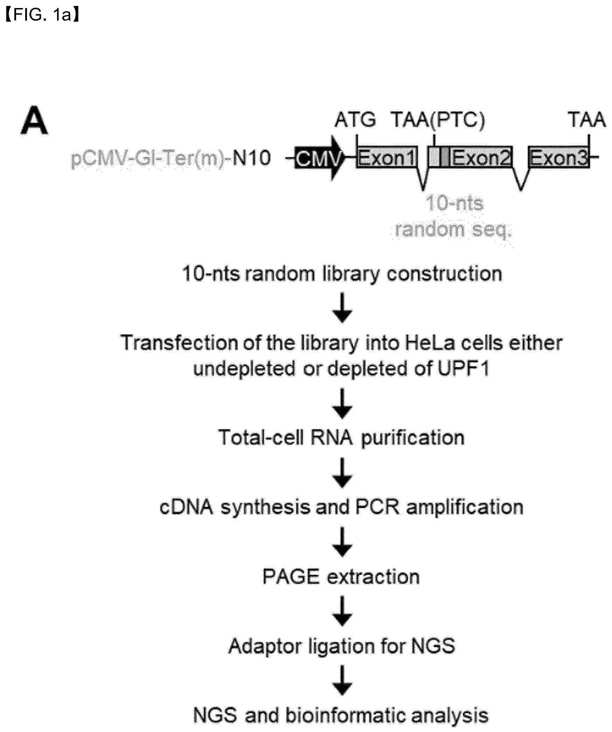

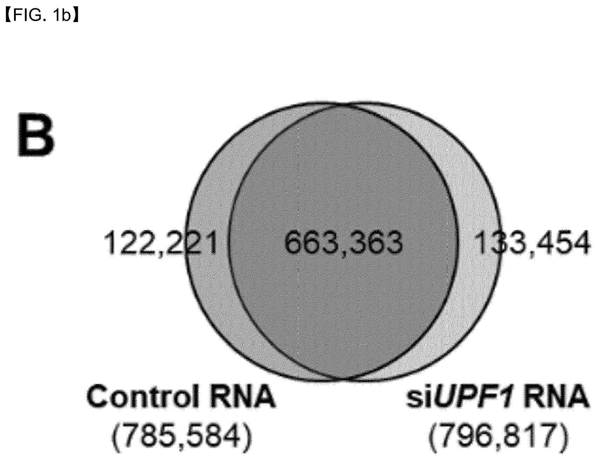

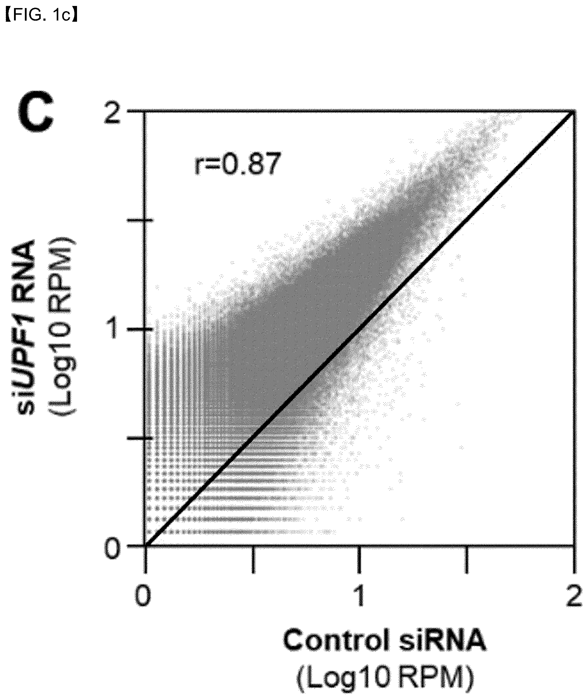

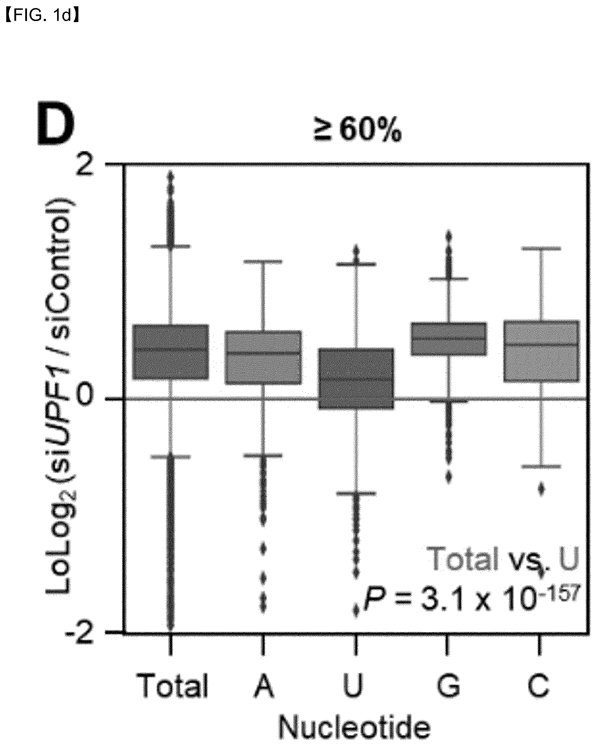

Figures 1a to 1i illustrate the results of base sequence screening using NMD reporter mRNA. Specifically, Figure 1a shows the experimental schematic diagram, and Figure 1b is a Venn diagram demonstrating that similar levels of reporter libraries were generated when treated with control siRNA and UPF1 siRNA. Figure 1c illustrates that most reporter mRNAs increase due to the inhibition of UPF1 expression. Figures 1d to 1i present analysis results showing that reporter mRNAs with U-rich sequences are independent of UPF1. Specifically, when the uridine content exceeds 60%, reporter mRNAs exhibit no dependence on UPF1.

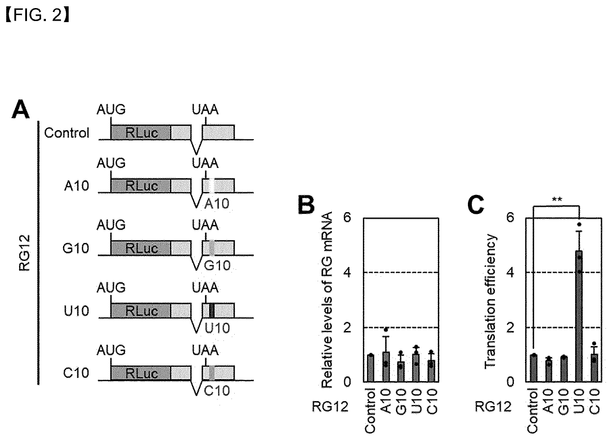

Figure 2 illustrates the results of experiments demonstrating selective translation increase depending on nucleotide type. Specifically, (A) illustrates the experimental schematic diagram, (B) illustrates qRT-PCR results indicating that the relative levels of reporter mRNA are not significantly different, and (C) illustrates results comparing protein translation efficiency, indicating that U10 increases protein translation efficiency by approximately 5-fold compared to A10, G10, and C10.

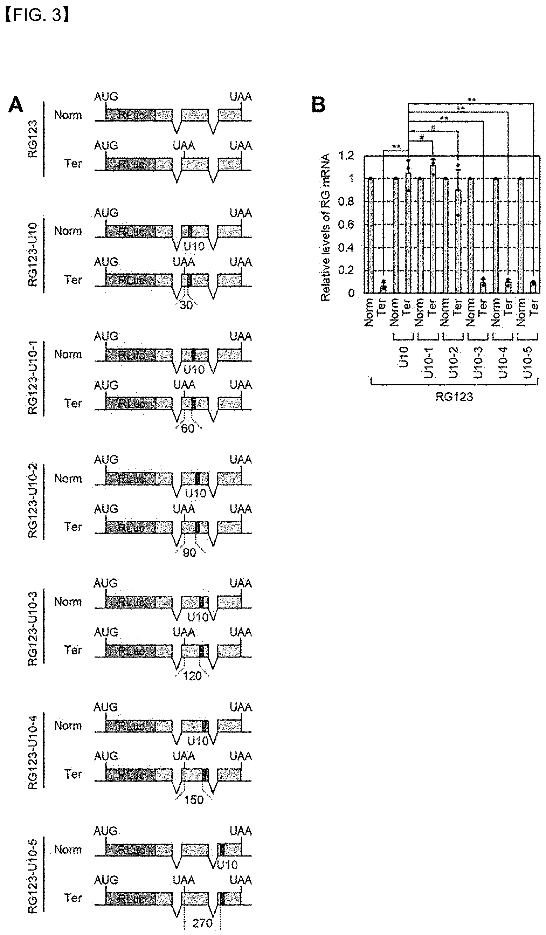

Figure 3 illustrates experimental results demonstrating the importance of the distance between the protein translation termination codon and U10 in the NMD reporter. Specifically, (A) illustrates the experimental schematic, and (B) illustrates the NMD efficiency in each reporter.

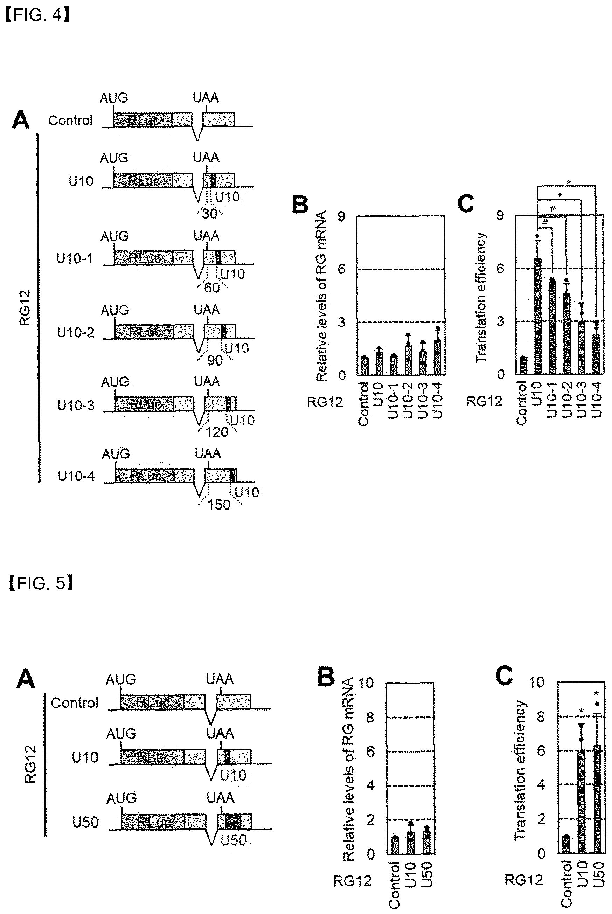

Figure 4 also illustrates experimental results demonstrating the importance of the distance between the protein translation termination codon and U10 in the NMD reporter. Specifically, (A) illustrates the experimental schematic, (B) illustrates qRT-PCR results indicating that the relative levels of reporter mRNA are not significantly different, and (C) illustrates results comparing protein translation efficiency, demonstrating that the distance between the protein translation termination codon and U10 effectively increases protein translation when the distance is 90 base pairs or less.
Figure 5 illustrates the results of experiments using reporters with consecutive U10 (U10) or U50 (U50) sequences. Specifically, (A) illustrates the reporter schematic diagram, (B) illustrates qRT-PCR results indicating no significant difference in the relative levels of reporter mRNA, and (C) illustrates results comparing protein translation efficiency, demonstrating that both U10 and U50 increase protein translation efficiency by approximately 6-fold.

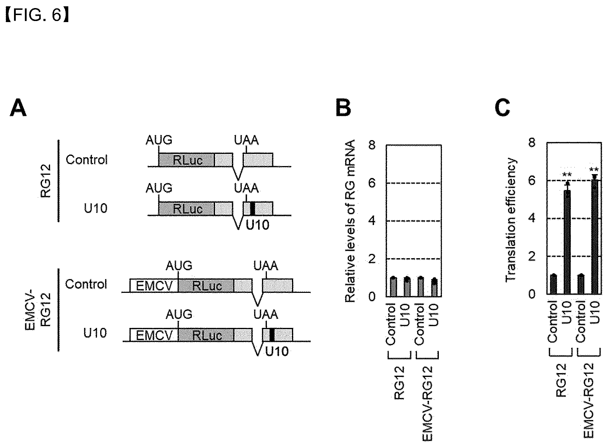

Figure 6 illustrates the results of verifying the protein translation efficiency of U10 using IRES. Specifically, (A) illustrates the reporter schematic diagram, (B) illustrates qRT-PCR results indicating no difference in the relative levels of reporter mRNA, and (C) illustrates the results of comparing protein translation efficiency, demonstrating that EMCV IRES increased protein translation efficiency by approximately 6-fold.

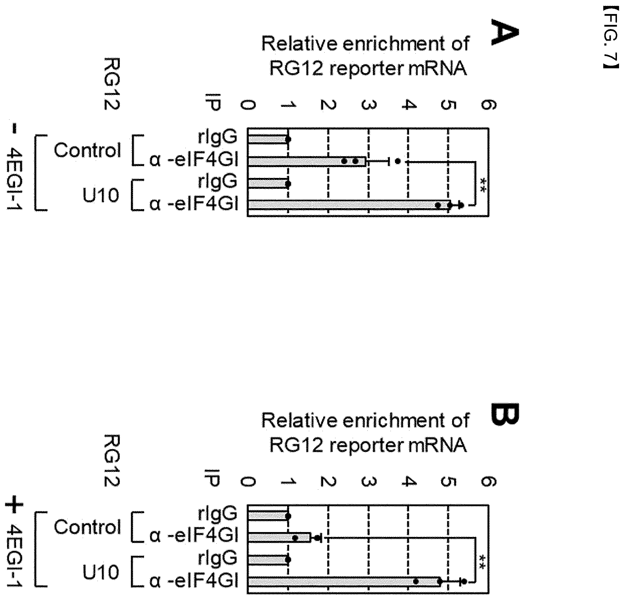

Figure 7 illustrates the experimental results demonstrating the binding between U10 reporter and eIF4G via eIF4G immunoprecipitation (IP). Specifically, (A) illustrates the results of eIF4G immunoprecipitation in the absence of 4EGI-1, and (B) illustrates the results of eIF4G immunoprecipitation after treatment with 4EGI-1.

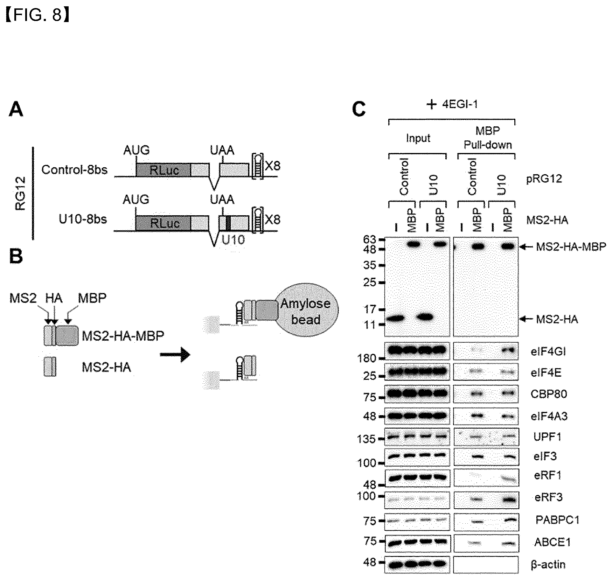

Figure 8 illustrates the experimental results demonstrating the specific binding between U10 reporter mRNA and the termination factor. Specifically, (A) is a schematic diagram of U10 reporter mRNA, (B) is a schematic diagram of MS2-HA-MBP used for purifying the reporter mRNA, and (C) illustrates the Western blot experimental results of various translation factors binding to the purified reporter mRNA.

### Detailed Description of Invention

Hereinafter, the present invention will be described in more detail by way of examples. These examples are only for describing the present invention in more detail, and it will be apparent to those skilled in the art that according to the gist of the present invention, the scope of the present invention is not limited by these examples.

### Example 1. Identification of nucleotide sequences affecting mRNA stability using an NMD reporter

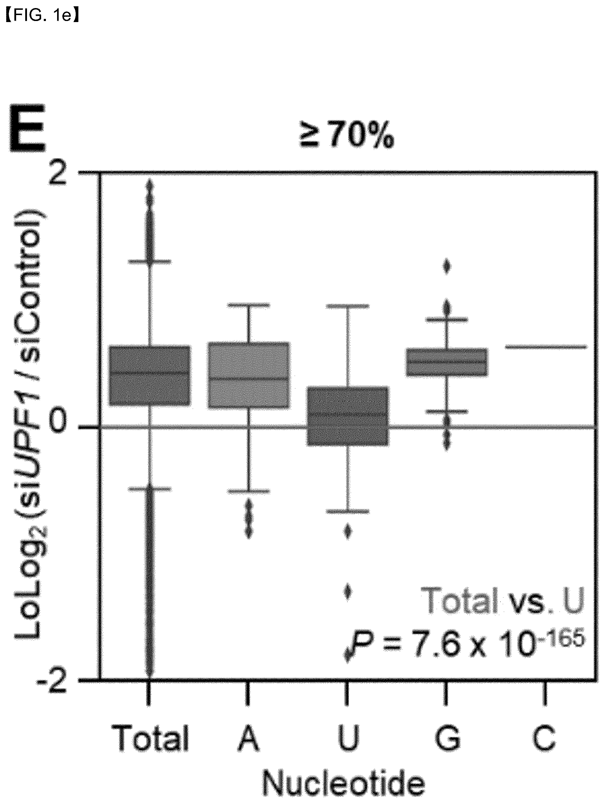

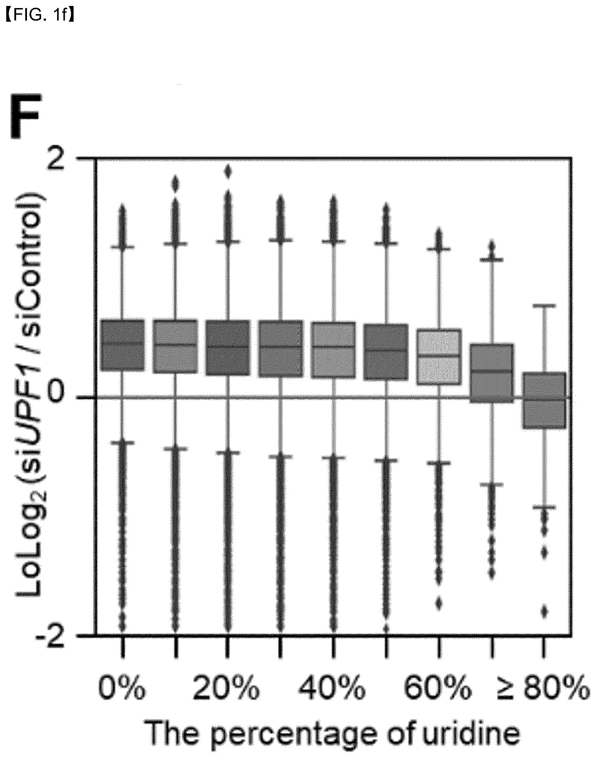

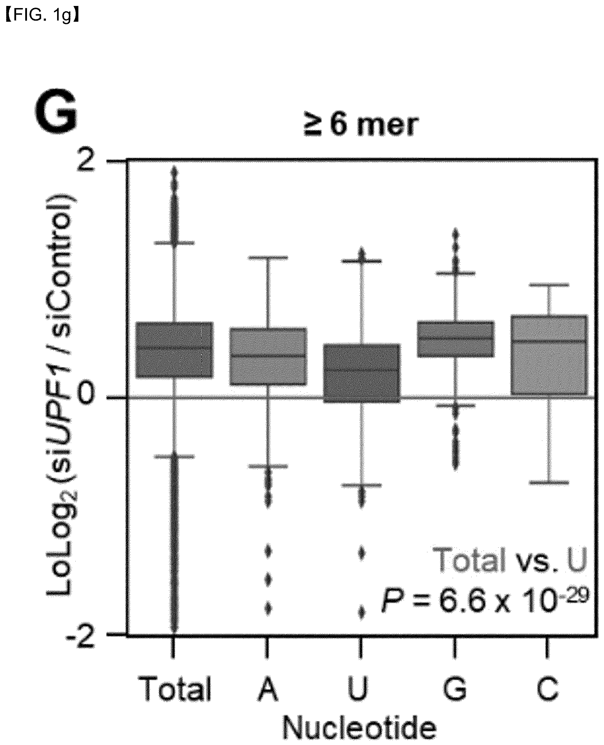

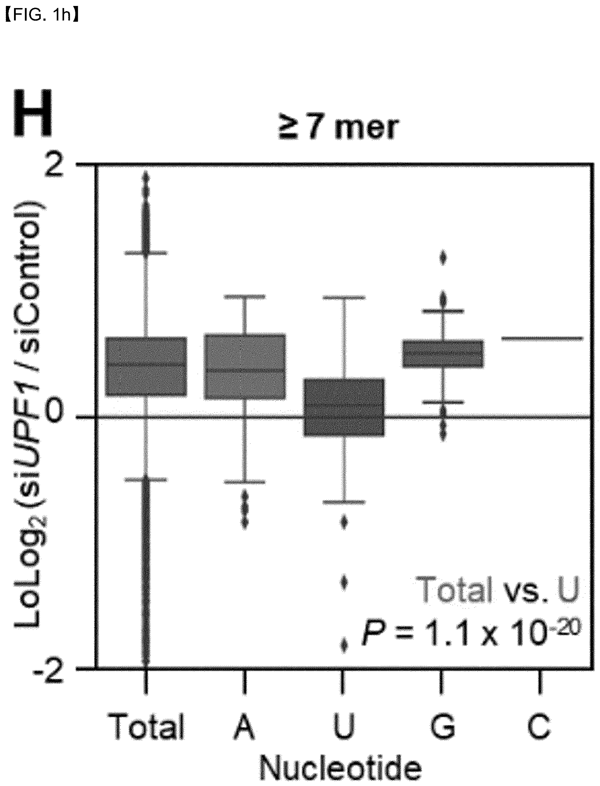

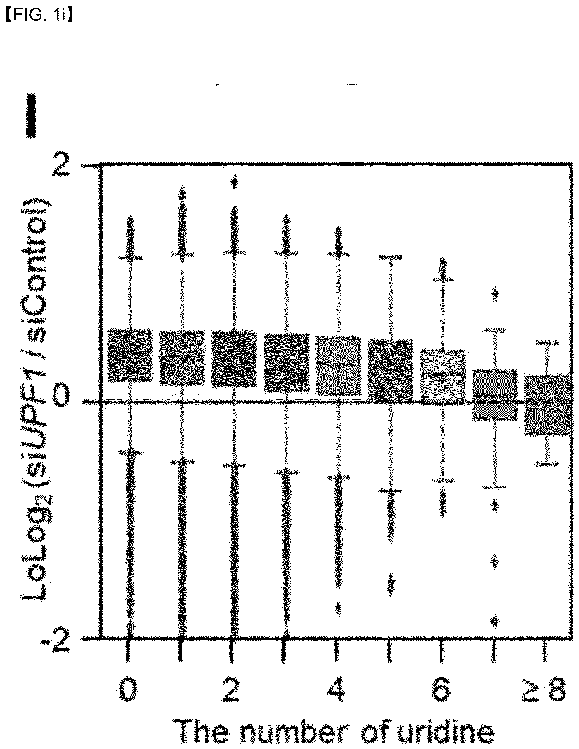

Nonsense-mediated mRNA decay (NMD) is a cellular phenomenon determined by protein translation efficiency, where the amount of NMD reporter mRNA decreases as translation efficiency increases. Using this, we created an NMD reporter mRNA with 10 consecutive random sequences immediately following the premature translation termination codon (PTC) of the NMD reporter mRNA, as shown in Figure 1.

In other words, we artificially created a reporter containing 4¹⁰ different nucleotide sequences immediately following the protein translation termination codon that triggers NMD. The constructed reporter mRNA was expressed in HeLa cells, and the stability of each reporter mRNA was investigated using next-generation sequencing (NGS) technology. Specifically, the efficiency of NMD was analyzed by comparing the presence of UPF1, a key factor in NMD, with its expression being inhibited.

The experimental results showed that most NMD reporter mRNAs increased in quantity upon UPF1 expression inhibition. However, reporter mRNAs with U-rich sequences showed no dependence on UPF1 (Regulator of nonsense transcripts 1 protein, encoded by the UPF1 gene). This result indicates that mRNAs with U-rich sequences following the translation termination codon are not targeted by NMD.

### Example 2. Identification of nucleotides that enhance protein translation efficiency among U, A, G, and C

Similarly, to directly monitor changes in translation efficiency caused by U-rich nucleotide sequences, a general mRNA reporter was used instead of an NMD reporter. After creating various reporters, experiments were conducted to identify which of the nucleotides U, A, G, and C located after the protein stop codon enhance mRNA translation efficiency.

As shown in Figure 2, when comparing U10, A10, G10, and C10 following the protein termination codon, the relative mRNA levels of U10, A10, G10, and C10 were similar; however, only U10 significantly improved mRNA translation efficiency.

### Example 3. Experiment on the importance of the distance between protein translation stop codons and U-rich nucleotide sequences

As such, after confirming whether U-rich nucleotide sequences influence mRNA stability, this experiment used NMD reporter mRNA composed of 10 consecutive Uridine sequences (U10) to examine the relative mRNA stability depending on the distance between the protein translation termination codon and U10.

As shown in Figure 3, the inhibition of NMD by the presence of U10 revealed that the distance between the protein translation termination codon and U10 is crucial. Specifically, efficient NMD inhibition was observed when the distance between the protein translation termination codon and U10 was 90 nucleotides or less, whereas the NMD inhibitory effect disappeared when the distance was 120 nucleotides or more. In other words, when the distance between the protein translation termination codon and U10 is 120 nucleotides or more, mRNA instability was confirmed.

Additionally, as shown in Figure 4, similar to the experimental results examined in Figure 3, it was confirmed that protein translation is effectively increased when the distance between the protein translation termination codon and U10 is 90 nucleotides or less.

### Example 4. Experiment on the efficiency of mRNA translation increase according to the length of U-rich nucleotide sequences

To directly monitor changes in translation efficiency caused by U-rich sequences, a general mRNA reporter was used instead of an NMD reporter. After creating various reporters, experiments were conducted to confirm whether mRNA translation efficiency differed depending on the length of the U-rich sequence present after the protein termination codon.

As shown in Figure 4, the U10 or U50 sequences located after the protein stop codon were found to increase protein translation efficiency by approximately 6-fold or more to a similar extent. Additionally, although not included in Figure 3, sequences longer than U100 were also found to enhance mRNA translation efficiency. Therefore, U-rich nucleotide sequences located after protein stop codons enhance mRNA-to-protein translation efficiency starting from U6, as shown in Figure 1, and there is no specific upper limit on the maximum length of U-rich nucleotide sequences that enhance mRNA translation efficiency.

### Example 5. Elucidation of the mechanism by which U-rich nucleotide sequences increase translation via the eIF4G translation factor

To determine which process of translation is affected by the increase in translation efficiency caused by U-rich nucleotide sequences, a reporter containing an IRES (internal ribosome entry site) was used to confirm the relative levels of mRNA and mRNA translation efficiency.

As shown in Figure 6, in the case of EMCV (encephalomyocarditis virus) IRES, an increase in mRNA translation induced by U10 was observed, resulting in approximately a 6-fold improvement in mRNA translation efficiency.

### Example 6. Analysis of eIF4G binding to mRNA containing the U10 sequence following the translation termination codon

Based on the finding that eIF4G is involved in translation via U10, we investigated whether eIF4G binds more effectively to mRNA containing the U10 sequence. eIF4G can exist at the 5' end of mRNA through its binding with eIF4E. To exclude this possibility, we compared samples treated with 4EGI-1 (EMD Chemicals, San Diego, CA) at 500 µM, which inhibits eIF4E-eIF4G binding, with untreated samples.

The experimental results, as shown in Figure 7, indicate that eIF4G antibody (a polyclonal antibody obtained from rabbit serum; reference paper: Molecular and Cellular Biology, Mar. 2005, pp. 2450-2462) was used to perform immunoprecipitation (IP) of eIF4G. Even in the presence of 4EGI-1, specific binding between eIF4G and reporter mRNA containing U10 was observed. This indicates that eIF4G binds directly or indirectly to U10. Even in the presence of 4EGI-1, specific binding between U10-containing reporter mRNA and eIF4G was observed. This indicates that eIF4G binds to U10.

### Example 7. mRNA containing the U10 sequence after the translation termination codon forms a more stable binding with the translation termination factors eRF1 and eRF3

To investigate the biomolecular mechanisms underlying the increased translation in U10-containing reporters, we purified the U10-containing reporter directly from cells and examined the translation factors that bind to it. To exclude protein-protein interactions mediated by eIF4E-eIF4G binding, 4EGI-1 compound was treated throughout all processes. As shown in the schematic diagram in Figure 8(A), an MS-binding sequence capable of binding to MS2-binding proteins was inserted into the 3'UTR of the U10 reporter mRNA. Concurrently, MS2-HA-MBP protein was expressed in cells. As shown in Figure 8(B), the expressed MS2-HA-MBP protein binds to the MS2-binding sequence of the reporter mRNA via MS2, and MBP forms a strong binding to MBP beads. Using this principle, the U10 reporter mRNA expressed in cells was purified, and the translation factors present in the purified sample were identified using Western blotting.

The experimental results, as shown in Figure 8(C), the translational termination factors eRF1 and eRF3 were relatively more abundant in reporter mRNA with U10 following the translation termination codon. This indicates that the presence of U10 promotes more active protein translation termination, thereby increasing protein translation efficiency.

## Claims

1. A nucleotide comprising a uridine-rich sequence in the 3' UTR (untranslated region) following the protein translation termination codon of the target nucleic acid sequence.

2. The nucleotide according to claim 1,
wherein the target nucleic acid sequence is a mRNA sequence.

3. The nucleotide according to claim 2,
wherein a distance between the protein translation termination codon of the target nucleic acid sequence and the uridine-rich sequence is 120 nt or less.

4. The nucleotide according to claim 3,
wherein a distance between the protein translation termination codon of the target nucleic acid sequence and the uridine-rich sequence is 105 nt or less.

5. The nucleotide according to claim 4,
wherein the length of the uridine-rich sequence is 6 nt or more.

6. The nucleotide according to claim 5,
wherein the length of the uridine-rich sequence is 10 nt or more.

7. The nucleotide according to any one of claims 1 to 6.
wherein the nucleotide is for increasing the protein translation efficiency of an RNA vaccine or therapeutic mRNA.

8. A method for increasing the protein translation efficiency of a target nucleic acid, comprising the step of inserting a uridine-rich sequence into the 3' UTR after the protein translation stop codon of the target nucleic acid sequence.

9. The method according to claim 8.
wherein the target nucleic acid sequence is an mRNA sequence.

10. The method according to claim 9.
wherein a distance between the protein translation stop codon of the target nucleic acid sequence and the uridine-rich sequence is 120 nt or less.

11. The method according to claim 10.
wherein a distance between the protein translation stop codon of the target nucleic acid sequence and the uridine-rich sequence is 105 nt or less.

12. The method according to claim 11.
wherein the length of the uridine-rich sequence is 6 nt or more.

13. The method according to claim 12.
wherein the length of the uridine-rich sequence is 10 nt or more.

14. The method according to any one of claims 8 to 13.
wherein the nucleotide is for increasing the protein translation efficiency of an RNA vaccine or therapeutic mRNA.

15. A vector for increasing the translation efficiency of the target protein, comprising a sequence encoding the target protein, and including uridine in the 3' UTR following the protein translation stop codon of the sequence encoding the target protein.

16. The vector according to claim 15.
wherein a distance between the protein translation stop codon of the sequence encoding the target protein and the uridine-rich sequence is 120 nt or less.

17. The vector according to claim 16.
wherein a distance between the protein translation stop codon of the sequence encoding the target protein and the uridine-rich sequence is 105 nt or less.

18. The vector according to claim 17.
wherein the length of the uridine-rich sequence is 6 nt or more.

19. The vector according to claim 18.
wherein the length of the uridine is 10 nt or more.

20. The vector according to any one of claims 15 to 19,
wherein the vector is a plasmid vector or a viral vector.

21. A cell line for increasing the translation efficiency of the target protein comprising the vector according to any one of claims 15 to 19.
